# EUROPEAN PATENT APPLICATION

(11) **EP 4 075 128 A1**
(43) Date of publication of application: **19.10.2022**
(21) Application number: 20897831.2
(22) Date of filing: 11.12.2020
(51) Int. Cl.: G01N 27/327

(54) **MICROELECTRODE OF GENE SEQUENCING CHIP, MANUFACTURING METHOD THEREFOR, AND GENE SEQUENCING CHIP**

(30) Priority: 13.12.2019 CN 201911283304
(71) Applicant: Geneus Technologies (Chengdu) Co., Ltd., Chengdu, Sichuan 610041 (CN); Dalian University of Technology, Dalian, Liaoning 116024 (CN)
(72) Inventor: SU, Yunpeng, Chengdu, Sichuan 610041 (CN); ZHOU, Dayu, Chengdu, Sichuan 610041 (CN); SUN, Nana, Chengdu, Sichuan 610041 (CN); GU, Jiaye, Chengdu, Sichuan 610041 (CN)
(74) Representative: Wenzel Nemetzade Warthmüller Patentanwälte Part mbB
(86) International application number: PCT/CN2020/135772
(87) International publication number: WO 2021/115436

(57) **Abstract**

Disclosed in the embodiments of the present application are a microelectrode of a gene sequencing chip, a manufacturing method therefor, and a gene sequencing chip. The microelectrode comprises a substrate, a current collector layer formed on the substrate, and an electrode layer formed on the current collector layer; the current collector layer comprises a transition metal thin film or a nitride thin film thereof or a composite thin film of a transition metal and nitride thereof, and the electrode layer comprises a nitrogen oxide thin film of the transition metal, which is formed on the transition metal thin film or the nitride thin film thereof or the composite thin film of the transition metal and nitride thereof. The embodiments of the present application improve the per unit area voltage driving capabilities of a microelectrode in a gene sequencing chip, can meet requirements for an ultra-high number of cycles, and improve the throughput and stability of a gene sequencing chip.

## Description

### TECHNICAL FIELD

The present invention relates to the technical field of a gene sequencing chip, especially a microelectrode of a gene sequencing chip, a manufacturing method therefor, and a gene sequencing chip containing the microelectrode.

### BACKGROUND

Supercapacitor materials are widely used in manufacturing a microelectrode in a biochip utilized in a gene sequencer, which have the characteristics of high power density, long cycle life, etc. A microelectrode is composed of two portions, i.e., an electrode and a current collector. The electrode charges and discharges through the energy storage mechanism to provide driving voltage for biochemical sequencing system. The collector transports electrons and is connected to a charge-discharge and measurement circuit, so as to provide charge-discharge current for the electrode and transmit a characteristic current signal of sequencing to a measurement chip.

At present, the commonly used microelectrodes in biochips utilized by a gene sequencer are mainly metal nitride electrodes deposited on a metal current collector made of Al, Au, Cu, etc. The research shows that these materials can exhibit a specific capacitance of more than 10 mF/cm² when measured by the low scanning speed C-V method. However, due to the high internal resistance of the electrode material itself and the high contact resistance between the electrode and the current collector, the specific capacitance will rapidly decrease with the increase of the voltage sweep speed, and will be less than 4 mF/cm² at a sweep speed greater than 0.1 V/s, thereby failing to ensure high specific capacitance and high charge-discharge rates at the same time. As a result, the voltage driving capacity per unit area is insufficient, and it is impossible to reduce the size of the microelectrode that meets the requirements to less than 5 microns, such that the number of sequencing units on a single chip exceeds ten million orders of magnitude.

### SUMMARY OF THE INVENTION

The embodiments of the present application provide a microelectrode of a gene sequencing chip, a manufacturing method therefor, and a gene sequencing chip, for solving the problems in the prior art, i.e., insufficient voltage driving capacity per unit area of a microelectrode in a gene sequencing chip.

In the first aspect, an embodiment of the present application provides a microelectrode of a gene sequencing chip, comprising:
a substrate;
a current collector layer formed on the substrate, which comprises a transition metal thin film or a nitride thin film thereof or a composite thin film of a transition metal and nitride thereof;
an electrode layer formed on the current collector layer, which comprises an oxynitride thin film of the transition metal formed on the transition metal thin film or the nitride thin film thereof or the composite thin film of the transition metal and the nitride thereof.

In an optional embodiment, the material of the substrate comprises Si, Ge, one of the III-V semiconductor materials, or glass, or ceramics.

In an optional embodiment, the III-V semiconductor materials comprise gallium arsenide.

In an optional embodiment, the transition metal comprises at least one of Ti, V, Ta, Mo and Hf.

In an optional embodiment, the transition metal thin film or the nitride thin film thereof or the composite thin film of the transition metal and the nitride thereof has a thickness of 10 ~ 5,000 nm.

In an optional embodiment, the transition metal thin film or the nitride thin film thereof or the composite thin film of the transition metal and the nitride thereof has a resistivity of less than 500 µΩ·cm.

In an optional embodiment, the oxynitride thin film of the transition metal has a thickness of 10∼5,000 nm.

In an optional embodiment, the oxynitride thin film of the transition metal has a resistivity of larger than 1,000 µΩ·cm.

In an optional embodiment, when the current collector layer comprises the nitride thin film of the transition metal or the composite thin film of the transition metal and the nitride thereof, the stoichiometric ratio of metal element to nitrogen element in the nitride is 0.9 ~ 1.1.

In an optional embodiment, in the oxynitride thin film of the transition metal, the nitrogen content is 15~49 mol%, and the oxygen content is 1~35 mol%.

In the second aspect, an embodiment of the present application provides a manufacturing method for a microelectrode of a gene sequencing chip, comprising:
providing a substrate;
depositing a transition metal thin film or a nitride thin film thereof or a composite thin film of a transition metal and nitride thereof on the substrate as a current collector layer;
depositing an oxynitride thin film of the transition metal on the transition metal thin film or the nitride thin film thereof or the composite thin film of the transition metal and the nitride thereof as an electrode layer.

In an optional embodiment, cleaning the surface of the substrate using a standard cleaning process is further included.

In an optional embodiment, depositing an oxynitride thin film of the transition metal on the transition metal thin film or the nitride thin film thereof or the composite thin film of the transition metal and the nitride thereof as an electrode layer comprises: continuously growing in situ, a layer of oxynitride thin film of the transition metal with rough surface, nanopores and high specific capacitance, on the transition metal thin film or the nitride thin film thereof or the composite thin film of the transition metal and the nitride thereof, as an electrode layer, by adjusting deposition process parameters.

In an optional embodiment, the material of the substrate comprises Si, Ge, one of III-V semiconductor materials, or glass, or ceramics.

In an optional embodiment, the III-V semiconductor materials comprise gallium arsenide.

In an optional embodiment, the transition metal thin film or the nitride thin film thereof or the composite thin film of the transition metal and the nitride thereof has a thickness of 10∼5,000 nm.

In an optional embodiment, the transition metal thin film or the nitride thin film thereof or the composite thin film of the transition metal and the nitride thereof has a resistivity of less than 500 µΩ·cm.

In an optional embodiment, the oxynitride thin film of the transition metal has a thickness of 10∼5,000 nm.

In an optional embodiment, the oxynitride thin film of the transition metal has a resistivity of larger than 1,000 µΩ·cm.

In an optional embodiment, when the current collector layer comprises the nitride thin film of the transition metal or the composite thin film of the transition metal and the nitride thereof, the stoichiometric ratio of metal element to nitrogen element in the nitride is 0.9 - 1.1.

In an optional embodiment, in the oxynitride thin film of the transition metal, the nitrogen content is 15~49 mol%, and the oxygen content is 1~35 mol%.

In the third aspect, the present application provides a gene sequencing chip, which comprises the microelectrode described in any embodiments as mentioned above.

In comparison with the prior art, through tailoring of material properties by changing deposition process parameters of thin films, the embodiments of the present application are able to achieve continuously growing in situ, a current collector layer, i.e., a transition metal thin film or a nitride thin film thereof or a composite thin film of a transition metal and nitride thereof with high conductivity, and an electrode layer, i.e., an oxynitride thin film of the transition metal with high specific capacitance. Therefore, the manufactured microelectrode can be provided with high specific capacitance and high charge-discharge rates at the same time, and the voltage driving capacity per unit area is improved. Further, the electrode material, i.e., oxynitride of a transition metal, can maintain high structural stability and electrochemical stability in the biochemical environment of sequencing, meet the requirements for an ultra-high number of cycles, and improve the throughput and stability of a gene sequencing chip. Moreover, the materials for manufacturing the microelectrode are common commercial materials with simple process, low cost, strong producibility and easy to achieve mass production.

### BRIEF DESCRIPTION OF THE DRAWINGS

The present invention will be more fully understood by the following detailed description in conjunction with the accompanying drawings, in which similar elements are numbered in a similar manner, wherein:
Fig. 1 shows a structural diagram of a microelectrode of a gene sequencing chip according to an embodiment of the present application;
Fig. 2 shows a schematic flowchart of a manufacturing method for a microelectrode of a gene sequencing chip according to an embodiment of the present application;
Fig. 3 shows a cyclic voltammogram of a TiNxOy single electrode manufactured according to Comparative Embodiment 1;
Fig. 4 shows a cyclic voltammogram of a VNxOy single electrode manufactured according to Comparative Embodiment 2;
Fig. 5 shows a cyclic voltammogram of a TiN current collector/TiNxOy microelectrode manufactured according to Embodiment 1 of the present application;
Fig. 6 shows a comparison chart of specific capacitances of two electrodes obtained according to Comparative Embodiment 1 and Embodiment 1 at different scanning rates;
Fig. 7 shows a comparison chart of the decay curves of constant voltage current with time of two electrodes obtained according to Comparative Embodiment 1 and Embodiment 1;
Fig. 8 shows a cyclic voltammogram of a VN current collector/VNxOy microelectrode manufactured according to Embodiment 2 of the present application;
Fig. 9 shows a comparison chart of specific capacitances of two electrodes obtained according to Comparative Embodiment 2 and Embodiment 2 at different scanning rates.

### DETAILED DESCRIPTION

The technical solutions of the present application will be clearly and completely described below through the embodiments and in conjunction with the accompanying drawings. However, the present application is not limited to the embodiments described below. Based on the following embodiments, all other embodiments obtained by those skilled in the art without creative efforts shall fall within the protection scope of the present application. For the sake of clarity, parts unrelated to describing the exemplary embodiments are omitted from the drawings.

It should be understood that terms such as "comprising" or "having" in this application are intended to indicate the presence of features, numbers, steps, acts, components or combinations thereof disclosed in this specification, and do not exclude the possibility that one or more other features, numbers, steps, behaviors, components or combinations thereof exist or are added.

As mentioned above, the microelectrode in a gene sequencing chip in the prior art is usually a metal nitride electrode deposited on a metal current collector made of Al, Au, and Cu, etc. The voltage driving capacity per unit area of this microelectrode material is insufficient and cannot meet the manufacturing requirement that the number of sequencing units in a single chip exceeds ten million orders of magnitude. With the study of the application of nitride (such as Tin, etc.), oxide (such as RuO₂, MnO₂, etc.) and oxynitride (such as TiNxOy, VNxOy, etc.) thin films of transition metals in the electrode materials of supercapacitors, the specific capacitance and resistivity can be controlled in a large range by adjusting the deposition process parameters and by adjusting the stoichiometric ratio of chemical components, crystallographic orientation, micro morphology, etc. In the microelectronics industry, TiN and TaN thin films with high conductivity are also used in the manufacturing of gate electrodes of transistors. Therefore, based on the controllable specific capacitance and resistivity of the oxynitride of the transition metal in a large range and the excellent conductivity of the transition metal or the nitride thereof, the present application proposes a new microelectrode for a gene sequencing chip and the manufacturing method therefor, which solve the problem of insufficient voltage driving capacity per unit area of traditional electrode materials in a gene sequencing chip, and significantly improve the throughput and stability of the gene sequencing chip.

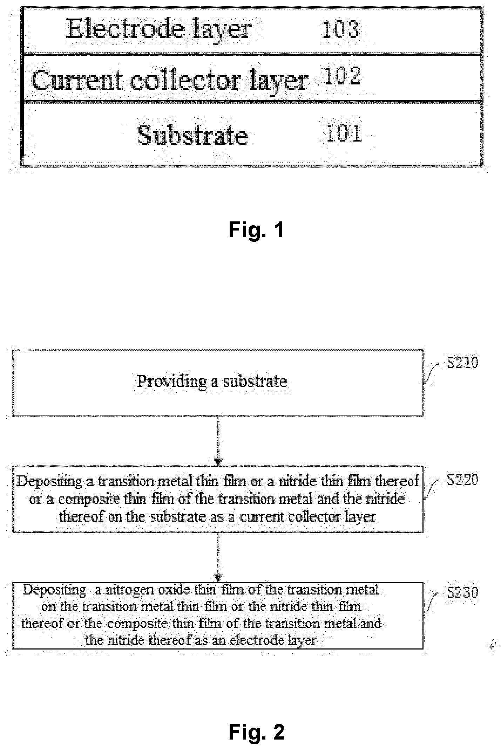

Fig. 1 shows a structural diagram of a microelectrode of a gene sequencing chip according to an embodiment of the present application. As shown in Fig. 1, the microelectrode of the gene sequencing chip according to the embodiment of the present application comprises: a substrate 101, a current collector layer 102 formed on the substrate 101 and an electrode layer 103 formed on the current collector layer 101.

The current collector layer 102 is formed by depositing a transition metal (M) thin film or a nitride (MN) thin film thereof or a composite (a composite of M and Mn) thin film of a transition metal and nitride thereof on the surface of the substrate 101. The electrode layer 103 is formed by depositing an oxynitride (MNxOy) thin film of the transition metal on the current collector layer 102.

By depositing an oxynitride thin film of the transition metal as an electrode layer on the highly conductive current collector layer of the transition metal thin film or the nitride thin film thereof or the composite thin film of the transition metal and the nitride thereof, high specific capacitance and high charge-discharge rates of a microelectrode are realized in the embodiments of the present application. The structure of this microelectrode can maintain high structural stability and electrochemical stability in the biochemical environment of sequencing, and can improve the throughput and stability of the gene sequencing chip.

In some embodiments, the material of the substrate 101 may include thesemiconductor material, such as silicon (Si) or germanium (Ge), or one of III-V semiconductor materials, such as gallium arsenide, and may also include the material for semiconductor manufacturing industries, such as glass substrates, ceramics, etc.

In some embodiments, the transition metal (M) may include at least one of titanium (Ti), vanadium (V), tantalum (Ta), molybdenum (Mo) and hafnium (Hf).

In some embodiments, the transition metal (M) thin film or the nitride (MN) thin film thereof or the composite thin film of the transition metal and the nitride thereof has a smooth surface, high density, low resistivity and high conductivity. As an example, the transition metal (M) thin film or the nitride (MN) thin film thereof or the composite thin film of the transition metal and the nitride thereof has a thickness of 10~5000 nm, and a resistivity of less than 500 µΩ·cm.

In some embodiments, the oxynitride thin film of the transition metal has a rough surface, exhibits a porous structure with nanopores, and has high resistivity, low conductivity and high specific capacitance. As an example, the oxynitride (MNxOy) thin film of the transition metal has a thickness of 10∼5,000 nm, and a resistivity of larger than 1,000 µΩ·cm.

In some embodiments, when the current collector layer 102 is formed with the nitride (MN) thin film of the transition metal or the composite (a composite of M and Mn) thin film of the transition metal and the nitride thereof, the stoichiometric ratio of metal element (M) to nitrogen element (N) in the nitride is 0.9 ~ 1.1, and the oxygen content is less than 15 mol%. The nitride thin film of the transition metal with a stoichiometric ratio close to 1:1 has excellent electrical conductivity.

In some embodiments, the composition of the oxynitride thin film of the transition metal is affected by factors such as film manufacturing conditions, the purity of the gas, the target and the precursor, and wherein the nitrogen content is 15~49 mol% and the oxygen content is 1~35 mol%.

FIG. 2 is a schematic flowchart of a manufacturing method for a microelectrode of a gene sequencing chip according to an embodiment of the present application. As shown in Fig. 2, the manufacturing method for a microelectrode of a gene sequencing chip according to an embodiment of the present application comprises the following steps:

Step S210, providing a substrate.

In this step, the substrate can be cleaned using a standard cleaning process first to remove impurities and dirt on the surface of the chip substrate. In some embodiments, the material of the substrate may include the semiconductor material such as silicon (Si) or germanium (Ge), or one of the III-V semiconductor materials, such as gallium arsenide, and may also include glass substrates. The substrate may have undergone other processes, such as forming patterns, structures, electrodes, etc., which are required for integrated circuits.

Step S220, depositing a transition metal thin film or a nitride thin film thereof or a composite thin film of a transition metal and nitride thereof on the substrate as a current collector layer.

In this step, the film deposition process may be utilized, effective regulation of the mechanism such as atomic diffusion and nucleation growth can be achieved by adjusting process parameters, and a layer of a transition metal (M) thin film or a nitride (MN) thin film thereof or a composite (a composite of M and Mn) thin film of a transition metal and nitride thereof, with smooth surface, high density and high conductivity (low resistivity), is deposited on the cleaned substrate as a current collector layer.

In some embodiments, the transition metal (M) may include at least one of titanium (Ti), vanadium (V), tantalum (Ta), molybdenum (Mo), and hafnium (Hf).

The process parameters include target-substrate distance, the ratio of argon to nitrogen, sputtering power, substrate temperature, working pressure, substrate bias voltage and other process parameters. It should be noted that the parameters may be different according to different process machines, and the differences of such process parameters do not affect the essence of the invention claimed in the present application, and are also covered by the protection scope of the embodiments of the present application.

In some embodiments, the film deposition process may include physical vapor deposition (PVD), chemical vapor deposition (CVD), or atomic layer deposition (ALD). Among them, the physical vapor deposition may include vacuum evaporation, sputtering coating, and arc plasma coating.

In some embodiments, when depositing the current collector layer by sputtering coating, a set of exemplary process parameters may be: target-substrate distance: 20~100 mm; Ar:N₂ = (10-200):(1-20) sccm; sputtering power: 100~10000 W; substrate temperature: room temperature to 400°C; working pressure: 0.2~2 Pa; substrate bias voltage: -50∼-400 V; and sputtering time: 0.5∼500 min.

In some embodiments, the transition metal thin film or the nitride thin film thereof or the composite thin film of the transition metal and the nitride thereof has a thickness of 10∼5,000 nm, and a resistivity of less than 500 µΩ·cm.

In some embodiments, when the current collector layer is formed with the nitride (MN) thin film of the transition metal or the composite (a composite of M and MN) thin film of the transition metal and the nitride thereof, the stoichiometric ratio of metal element (M) to nitrogen element (N) in the nitride is 0.9 ~ 1.1, and the oxygen content is less than 15 mol%. The nitride thin film of the transition metal with a stoichiometric ratio close to 1:1 has excellent electrical conductivity.

Step S230, depositing an oxynitride thin film of the transition metal on the transition metal thin film or the nitride thin film thereof or the composite thin film of the transition metal and the nitride thereof as an electrode layer.

In this step, by adjusting the deposition process parameters and changing growth conditions for the film to achieve certain crystallographic orientation and micro morphology, a layer of an oxynitride (MNxOy) thin film of the transition metal, with rough surface, porous structure, low conductivity (high resistivity) and high specific capacitance, is continuously grown in situ on the current collector layer of the transition metal (M) thin film or the nitride (MN) thin film thereof or the composite (M and MN composite) thin film of the transition metal and the nitride thereof, as an electrode layer. The electrode layer of the oxynitride (MNxOy) of the transition metal can be patterned by means of photolithography or mask after passing the physical and chemical property test.

In some embodiments, when depositing the electrode layer by sputtering coating, a set of exemplary process parameters may be: target-substrate distance: 20~100 mm; Ar:N₂ = (10-200):(1-20) sccm; sputtering power: 100~10000 W; substrate temperature: room temperature to 400°C; working air pressure: 0.4~2 Pa; and sputtering time: 0.5~500 min.

In some embodiments, the oxynitride thin film of the transition metal has a thickness of 10~5000 nm, and a resistivity of larger than 1000 µΩ·cm.

In some embodiments, the composition of the oxynitride thin film of the transition metal is affected by factors such as film manufacturing conditions, the purity of gas, the target and the precursor, and wherein the nitrogen content is 15~49 mol% and the oxygen content is 1~35 mol%.

The following is a comparative description based on the TiNxOy single electrode and the VNxOy single electrode respectively manufactured according to Comparative Embodiment 1 and Comparative Embodiment 2, and the microelectrodes respectively manufactured according to Embodiment 1 and Embodiment 2 of the present application.

### Comparative Embodiment 1:

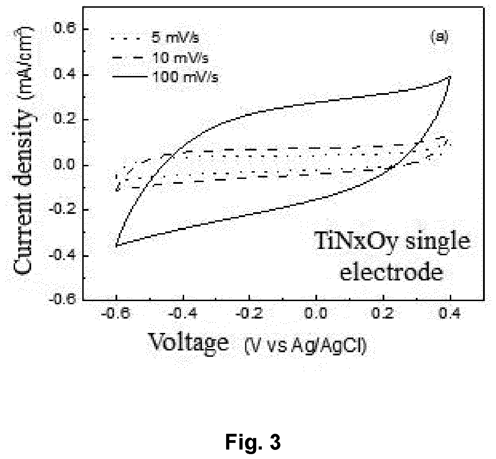

In this comparative embodiment, a monocrystalline silicon wafer is utilized as a substrate, and the substrate is cleaned by the standard RCA cleaning process in the semiconductor industry. A layer of titanium oxynitride (TiNxOy) single electrode, with a thickness of 200 nm, a porous structure and a resistivity of 2,000 µΩ·cm, is grown on the surface of the substrate, by adopting DC reactive magnetron sputtering, wherein the target material is titanium, and the process parameters are: target-substrate distance: 30 mm, Ar:N₂ = 10:1.1 sccm, sputtering power: 150 W, substrate temperature: 200°C, working pressure: 0.5 Pa, and puttering time: 20 min. By using the three-electrode test system, wherein the working electrode is TiNxOy, the counter electrode is a platinum electrode, the reference electrode is Ag\AgCl, and the electrolyte is KCl solution, a cyclic voltammetry curve is tested by the electrochemical workstation, as shown in Fig. 3.

### Comparative Embodiment 2:

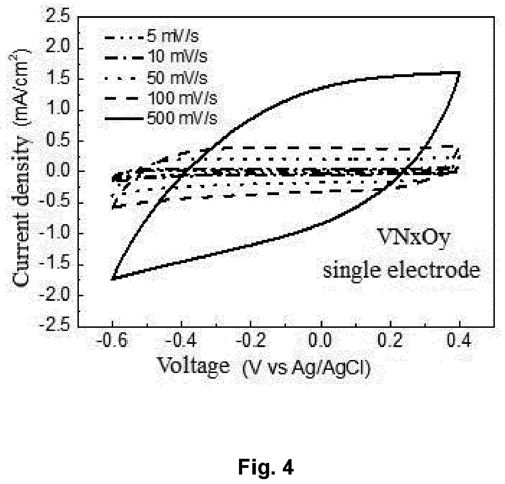

In this comparative embodiment, a monocrystalline silicon wafer is utilized as a substrate, and the substrate is cleaned by the standard RCA cleaning process in the semiconductor industry. A layer of porous VNxOy electrode with a thickness of 220nm and a resistivity of 3,200 µΩ·cm is grown on the surface of the substrate, by adopting DC reactive magnetron sputtering, and wherein the target material is vanadium, and the process parameters are: target-substrate distance: 50 mm, Ar:N₂=15:1.5 sccm, sputtering power: 250 W, substrate temperature: 300°C, working pressure: 0.6 Pa, and sputtering time: 20 min. By using the three-electrode test system, wherein the working electrode is VNxOy, the counter electrode is a platinum electrode, the reference electrode is Ag\AgCl, and the electrolyte is KCl solution, a cyclic voltammetry curve is tested by the electrochemical workstation, as shown in Fig. 4.

### Embodiment 1:

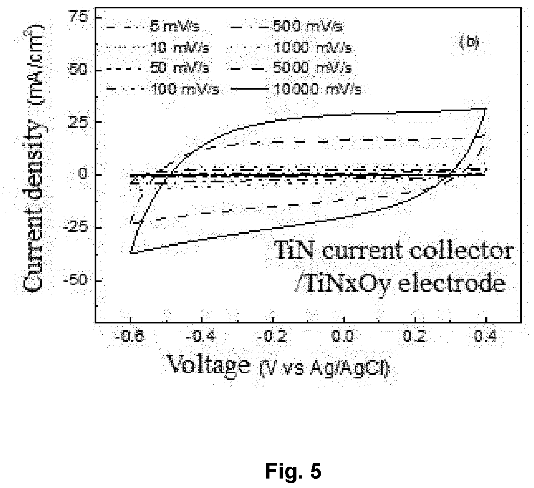

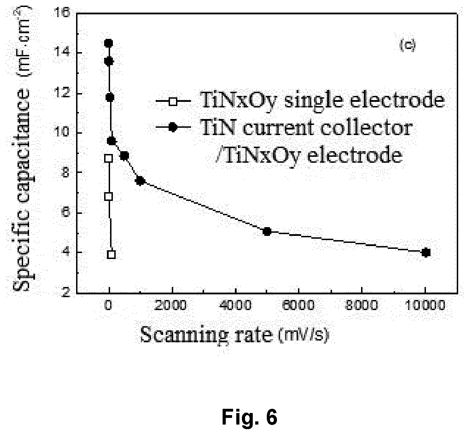

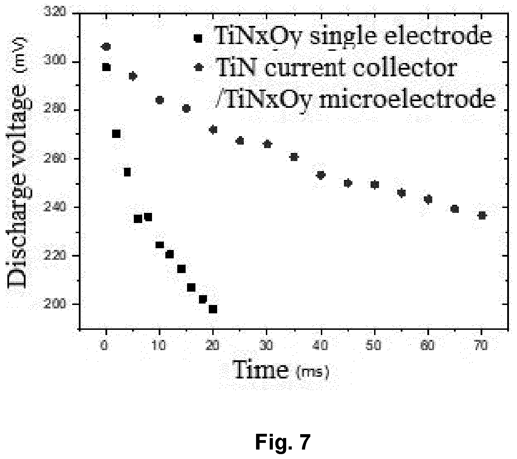

In this embodiment, a monocrystalline silicon wafer is utilized as a substrate, and the substrate is cleaned by the standard RCA cleaning process in the semiconductor industry. A layer of TiN thin film, with a thickness of 58nm, a resistivity of 88µΩ·cm, a smooth surface and high density, is deposited on the surface of the substrate as the current collector layer, by adopting DC reactive magnetron sputtering, wherein the target material is titanium, and the process parameters are: target-substrate distance: 30 mm, Ar:N₂=10:1.1 sccm, sputtering power: 130 W, substrate temperature: 100°C, working pressure: 0.3 Pa, substrate bias voltage: -50 V, and the sputtering time: 15 min. Then, by adjusting the deposition process parameters, a TiNxOy film with a thickness of 200nm, a resistivity of 2000 µΩ·cm, a rough surface and nanopores is grown in situ on the TiN film as an electrode layer, wherein the process parameters are: target-substrate distance: 30 mm, Ar:N₂ = 10:1.1 sccm, sputtering power: 150 W, substrate temperature: 200°C, working pressure: 0.5 Pa, and sputtering time: 20 min. By using the three-electrode test system, wherein the working electrode is TiNxOy, the counter electrode is a platinum electrode, the reference electrode is Ag\AgCl, and the electrolyte is KCl solution, a cyclic voltammetry curve is tested by the electrochemical workstation, as shown in Fig. 5. As can be seen from Fig. 5, the curve has good rectangularity at high scanning speed, which indicates that the TiN current collector\TiNxOy electrode manufactured in this embodiment has small internal resistance and good rate capability. As shown in Fig. 6, compared with the TiNxOy single electrode in Comparative Embodiment 1, the specific capacitance of the TiN current collector/TiNxOy electrode manufactured in Embodiment 1 increases to 14.5 mF/cm² from the original 8.7 mF/cm²; and the maximum scanning rate increases to 10000mV/s from the original 100 mV/s, and the specific capacitance is still as high as 4 mF/cm² at the scanning rate of 1,0000 mV/s. Using the transient measurement method by controlling the potential step, the current response curve is tested by standard measurement methods, and the current decay time is measured as shown in Fig. 7, wherein the working electrode is TiNxOy, the counter electrode is an Ag\AgCl electrode, the electrolyte is KCl solution, and a 20 KΩ series resistance is used. It can be seen from Fig. 7 that the discharge voltage dropping rate of the TiN current collector\TiNxOy electrode manufactured in this embodiment is significantly lower than that of the TiNxOy single electrode in the comparative embodiments, and the time to drop to 80% of the initial voltage is increased to about 60 ms compared with about 8 ms of the TiNxOy single electrode, and the voltage driving ability is greatly improved.

### Embodiment 2:

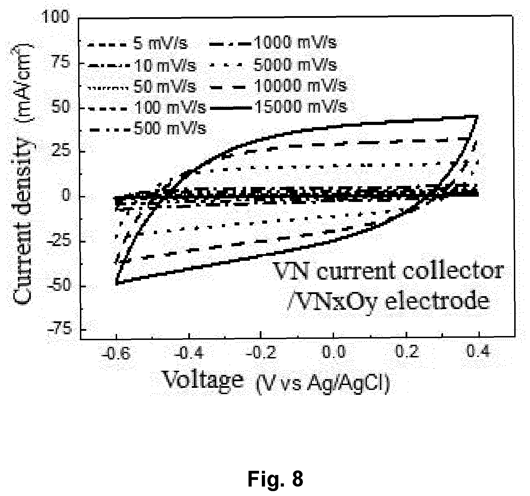

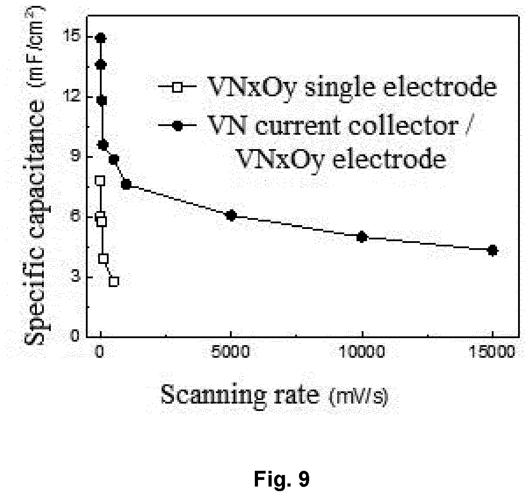

In this embodiment, a monocrystalline silicon wafer is utilized as a substrate, and the substrate is cleaned by the standard RCA cleaning process in the semiconductor industry. A layer of VN film, with a smooth surface, high density and high conductivity (low resistivity), is deposited on the surface of the cleaned substrate as the current collector layer, by adopting RF reactive magnetron sputtering, wherein the target material is vanadium, and the process parameters are: target-substrate distance: 20~100 mm, Ar:N₂ = (10-60):(1-10)sccm, sputtering power: 100~400 W, substrate temperature: room temperature to 400°C, working pressure: 0.2~2 Pa, substrate bias voltage: -50 - -400 V, and sputtering time: 5~500 min. Then, by adjusting the deposition process parameters, a VNxOy film with a rough surface, nanopores and low conductivity (high resistivity) is grown in situ on the VN film as an electrode layer, wherein the process parameters are: target-substrate distance: 20~100 mm, Ar:N₂ = (10-60):(1-10) sccm, sputtering power: 100~400 W, substrate temperature: room temperature to 400°C, working pressure: 0.4~2 Pa, and sputtering time: 5~500 min. By using the three-electrode test system, a cyclic voltammetry curve is tested by the electrochemical workstation, as shown in Fig. 8, wherein the working electrode is VNxOy, the counter electrode is a platinum electrode, the reference electrode is Ag\AgCl, and the electrolyte is KCl solution. As can be seen from Fig. 8, the curve has good rectangularity at high scanning speed, which indicates that the VN current collector\VNxOy electrode manufactured in this embodiment has small internal resistance and good rate capability. As shown in Fig. 9, compared with the VNxOy single electrode in Comparative Embodiment 2, the specific capacitance of the VN current collector/VNxOy electrode manufactured in Embodiment 2 increases to 14.9 mF/cm² from the original 7.8mF/cm²; and the maximum scanning rate increases to 15000 mV/s from the original 100 mV/s, and the specific capacitance is still as high as 4 mF/cm² at the scanning rate of 15000 mV/s.

An embodiment of the present application also provides a gene sequencing chip, which includes the microelectrode mentioned in any embodiment of the present application as described above.

The electrode of a gene sequencing chip and the manufacturing method therefor according to the embodiments of the present application overcome the defects of the traditional microelectrodes, i.e., being unable to be provided with high specific capacitance and high charge-discharge rates at the same time, which results in insufficient voltage driving capacity per unit area. In addition, the electrode material, i.e., the oxynitride of a transition metal, can maintain high structural stability and electrochemical stability in the biochemical environment of sequencing, meet the requirements for an ultra-high number of cycles, and improve the throughput and stability of the gene sequencing chip. Moreover, the materials for manufacturing the microelectrode are common commercial materials with simple process, low cost, strong producibility and easy to achieve mass production.

The embodiments of the present application are not limited to the those described in the above-mentioned embodiments. Without departing from the spirit and scope of the present application, those skilled in the art can make various changes and improvements to the present application in form and detail, all of which are considered to fall within the protection scope of the present application.

## Claims

1. A microelectrode of a gene sequencing chip, comprising:
a substrate;
a current collector layer formed on the substrate, comprising a transition metal thin film or a nitride thin film thereof or a composite thin film of a transition metal and nitride thereof;
an electrode layer formed on the current collector layer, comprising an oxynitride thin film of the transition metal formed on the transition metal thin film or the nitride thin film thereof or the composite thin film of the transition metal and the nitride thereof.

2. The microelectrode of a gene sequencing chip according to claim 1, wherein the material of the substrate comprises Si, Ge, one of III-V semiconductor materials, or glass, or ceramics.

3. The microelectrode of a gene sequencing chip according to claim 2, wherein the III-V semiconductor materials comprise gallium arsenide.

4. The microelectrode of a gene sequencing chip according to claim 1, wherein the transition metal comprises at least one of Ti, V, Ta, Mo and Hf.

5. The microelectrode of a gene sequencing chip according to claim 1, wherein the transition metal thin film or the nitride thin film thereof or the composite thin film of the transition metal and the nitride thereof has a thickness of 10~5,000 nm.

6. The microelectrode of a gene sequencing chip according to claim 5, wherein the transition metal thin film or the nitride thin film thereof or the composite thin film of the transition metal and the nitride thereof has a resistivity of less than 500 µΩ·cm.

7. The microelectrode of a gene sequencing chip according to claim 1, wherein the oxynitride thin film of the transition metal has a thickness of 10~5,000 nm.

8. The microelectrode of a gene sequencing chip according to claim 7, wherein the oxynitride thin film of the transition metal has a resistivity of larger than 1,000 µΩ·cm.

9. The microelectrode of a gene sequencing chip according to claim 1, wherein when the current collector layer comprises the nitride thin film of the transition metal or the composite thin film of the transition metal and the nitride thereof, the stoichiometric ratio of metal element to nitrogen element in the nitride is 0.9 ~ 1.1.

10. The microelectrode of a gene sequencing chip according to claim 1, wherein in the oxynitride thin film of the transition metal, the nitrogen content is 15-49 mol%, and the oxygen content is 1-35 mol%.

11. A manufacturing method for a microelectrode of a gene sequencing chip, comprising:
providing a substrate;
depositing a transition metal thin film or a nitride thin film thereof or a composite thin film of a transition metal and nitride thereof on the substrate as a current collector layer;
depositing an oxynitride thin film of the transition metal on the transition metal thin film or the nitride thin film thereof or the composite thin film of the transition metal and the nitride thereof as an electrode layer.

12. The manufacturing method for a microelectrode of a gene sequencing chip according to claim 11, further comprising:
cleaning a surface of the substrate using a standard cleaning process.

13. The manufacturing method for a microelectrode of a gene sequencing chip according to claim 11, wherein depositing an oxynitride thin film of the transition metal on the transition metal thin film or the nitride thin film thereof or the composite thin film of the transition metal and the nitride thereof as an electrode layer comprises:
continuously growing in situ a layer of oxynitride thin film of the transition metal with rough surface, nanopores and high specific capacitance, on the transition metal thin film or the nitride thin film thereof or the composite thin film of the transition metal and the nitride thereof by adjusting deposition process parameters.

14. The manufacturing method for a microelectrode of a gene sequencing chip according to claim 11, wherein the material of the substrate comprises Si, Ge, one of III-V semiconductor materials, or glass, or ceramics.

15. The manufacturing method for a microelectrode of a gene sequencing chip according to claim 14, wherein the III-V semiconductor materials comprise gallium arsenide.

16. The manufacturing method for a microelectrode of a gene sequencing chip according to claim 11, wherein the transition metal thin film or the nitride thin film thereof or the composite thin film of the transition metal and the nitride thereof has a thickness of 10~5,000 nm.

17. The manufacturing method for a microelectrode of a gene sequencing chip according to claim 16, wherein the transition metal thin film or the nitride thin film thereof or the composite thin film of the transition metal and the nitride thereof has a resistivity of less than 500 µΩ·cm.

18. The manufacturing method for a microelectrode of a gene sequencing chip according to claim 11, wherein the oxynitride thin film of the transition metal has a thickness of 10~5,000 nm.

19. The manufacturing method for a microelectrode of a gene sequencing chip according to claim 18, wherein the oxynitride thin film of the transition metal has a resistivity of larger than 1,000 µΩ·cm.

20. The manufacturing method for a microelectrode of a gene sequencing chip according to claim 11, wherein when the current collector layer comprises the nitride thin film of the transition metal or the composite thin film of the transition metal and the nitride thereof, the stoichiometric ratio of metal element to nitrogen element in the nitride is 0.9 ~ 1.1.

21. The manufacturing method for a microelectrode of a gene sequencing chip according to claim 11, wherein in the oxynitride thin film of the transition metal, the nitrogen content is 15-49 mol%, and the oxygen content is 1-35 mol%.

22. A gene sequencing chip, comprising the microelectrode according to any one of claims 1-10.
